Europäisches Patentamt

European Patent Office

Office européen des brevets

(19)

(11) Veröffentlichungsnummer : **0 034 293**
**B1**

(12) **EUROPÄISCHE PATENTSCHRIFT**

(45) Veröffentlichungstag der Patentschrift :
04.07.84

(21) Anmeldenummer : **81100721.0**

(22) Anmeldetag : **02.02.81**

(51) Int. Cl.³ : **C 12 N 11/04, C 12 N 11/12**

(54) **Verfahren zur Herstellung von Biokatalysatoren mit hoher mechanischer Festigkeit und hoher Beladung an enzymatisch aktiver Substanz und perlförmiger Biokatalysator.**

(30) Priorität : **15.02.80 DE 3005632**

(43) Veröffentlichungstag der Anmeldung :
**26.08.81 Patentblatt 81/34**

(45) Bekanntmachung des Hinweises auf die Patenterteilung : **04.07.84 Patentblatt 84/27**

(84) Benannte Vertragsstaaten :
**AT BE CH FR GB IT LI LU NL SE**

(56) Entgegenhaltungen :
EP-A- 0 033 397
CHEMICAL ABSTRACTS, Band 90, Nr. 21, 21. Mai
1979, Seite 221, Nr. 164051b Columbus, Ohio, U.S.A.
CHEMICAL ABSTRACTS, Band 90, Nr. 21, 21. Mai
1979, Seite 221, Nr. 164052c Columbus, Ohio, U.S.A.
CHEMICAL ABSTRACTS, Band 90, Nr. 21, 21. Mai
1979, Seite 221, Nr. 164053d Columbus, Ohio, U.S.A.
CHEMICAL ABSTRACTS, Band 81, Nr. 6, 12. August
1974, Seite 111, Nr. 27385p Columbus, Ohio, U.S.A.
M.S. MASRI et al.: "Binding of metal cations by
natural substances"
DIE ANGEWANDTE MAKROMOLEKULARE CHEMIE,
76/77, 1979, 329-350, Nr. 1141 J. KLEIN et al.:
"Polymernetzwerke zum Einschluss von Mikroorganismen"

(73) Patentinhaber : **Klein, Joachim,Prof. Dr.**
**Hühnerkamp 21**
**D-3300 Braunschweig (DE)**

**Wagner, Fritz, Prof. Dr.**
**Hohe Wiese 2**
**D-3300 Braunschweig-Stöckheim (DE)**

(72) Erfinder : **Klein, Joachim Prof. Dr.**
**Hühnerkamp 21**
**D-3300 Braunschweig (DE)**
Erfinder : **Wagner, Fritz Prof. Dr.**
**Hohe Wiese 2**
**D-3300 Braunschweig-Stöckheim (DE)**
Erfinder : **Vorlop, Klaus-Dieter Dipl.-Chem.**
**Hochstrasse 7**
**D-3300 Braunschweig (DE)**

(74) Vertreter : **Jahn-Held, Wilhelm W., Dr. Dr.-Ing. Dipl. Chem.**
**Schöne Aussicht 8**
**D-3513 Staufenberg 1 (DE)**

## Beschreibung

Die Fixierung von Enzymen, Zellfragmenten und ganzen Zellen hat in jüngster Zeit in der Fachwelt besondere Aufmerksamkeit gefunden. Dies liegt daran, dass solche Verfahren für die industrielle, biokatalytische Produktion von technischer und wirtschaftlicher Bedeutung ist.

Das Verfahren der nicht vorveröffentlichten, europäischen Patentanmeldung Nr. 0 033 398 (80200089.3) betrifft ein Verfahren zur Herstellung von Biokatalysatoren mit einer Druckfestigkeit (P/Perle) von über 800 bis 1 000 mit einer Beladung in Gramm enzymatischer Substanz pro Gramm Katalysatorperle bis 0,9.

Dieses Verfahren verwendet zur Lösung der ähnlichen Aufgabe als Fällungsbad (B) eine Lösung von Verbindungen mehrwertiger, dem Polyelektrolyten der Mischung (A) entgegengesetzt geladener, Ionen in einer Konzentration von 0,5 bis 35 Gew.-% in Wasser.

Die Fortenwicklung des in der Druckfestigkeit nicht begrenzten Verfahrens der Erfindung erweitert den unteren Konzentrationsbereich auf 0,02 Gew.-%. Das Verfahren nach dem Stand der Technik erzeugt in Stufe 1 perlförmige Teilchen im Kornbereich von 0,5 bis 5 mm.

Die Fortentwicklung des Verfahrens der Erfindung erweitert den oberen Kornbereich auf bis 10 mm. Das Verfahren nach dem Stand der Technik verwendet zur Nachhärtung das Fällungsbad aus Stufe 1. Dagegen verwendet das Verfahren der Erfindung in Stufe 4 zur Äquilibrierung eine, das Enzym-System und das Netzwerk stabilisierende wässrige Lösung. Eine solche besteht vorzugsweise aus einer Lösung oder Emulsion eines Schutzkolloides wie einer Lösung von Glycerin in Wasser in einer Konzentration von 1 bis 20 Gew.-%.

Die Fortentwicklung dieses Standes der Technik bietet den Vorteil einer grossen Breite in der Variation der Polymerkomponente und des Vernetzers.

Es ist die Aufgabe des Verfahrens der Erfindung, das Verfahren der europäischen Patentanmeldung Nr. 033398 weiterzuentwickeln. Die Aufgabe des Verfahrens der Erfindung ist im Oberbegriff des Anspruches 1 beschrieben.

Die Lösung der Aufgabe des Verfahrens der Erfindung ist im kennzeichnenden Teil des Anspruches 1 definiert.

Diese Lösung betrifft die Erweiterung des Konzentrationsbereiches der Lösung des Salzes in dem Fällungsbad (B), die Erweiterung des Kornbereiches der perlförmigen Teilchen, sowie das Einbringen der Katalysatorperlen in eine, das Enzym-System und das Netzwerk stabilisierende, wässrige Lösung oder Emulsion eines Schutzkolloides.

Die alternative Ausgestaltung des erfindungsgemässen Verfahrens ist in den Unteransprüchen definiert. Diese betreffen bevorzugt Formen des positiv oder negativ geladenen Polyelektrolyten in der Mischung (A), ionische Verbindungen in der Mischung (B), sowie die enzymatisch aktive Substanz und die Lösung oder Emulsion des Schutzkolloides in den Verfahrensstufen 2 und 4.

Das erfindungsgemässe Verfahren wird durch die folgenden Ausführungsbeispiele beschrieben. Dieses ist jedoch auf diese Beispiele, insbesondere in Bezug auf die enzymatisch aktive Substanz, nicht beschränkt.

Beispiel 1

Es wird die Mischung (A) durch Suspension von 30 g E-coli, ATCC 11105, in Form der zentrifugenfeuchten Masse in 570 ml einer 2,6 Gew.-%-igen, wässrigen Chitosan-acetat-Lösung als Polyelektrolyt hergestellt.

Als Fällungsbad (B) mit der Verbindung, die mehrwerige Ionen enthält, die dem Polyelektrolyten der Mischung (A) entgegengesetzt geladen sind, wird eine 0,05 molare $K_4$ $(Fe(CN)_6)$-Lösung verwendet.

Es wird dann in Stufe 1 die Mischung (A) in die Mischung (B) aus Kapillaren mit einem Durchmesser von 0,4 mm unter Rühren eingetropft, wobei perlförmige Teilchen mit einem Durchmesser von 4 mm entstehen. Diese werden unter weiterem Rühren bei einer Verweilzeit von 30 min. verfestigt. Es werden danach in Stufe 2 die gebildeten Katalysatorenperlen mit der eingeschlossenen enzymatisch aktiven Substanz abfiltriert und mit Wasser gewaschen.

Es werden danach in Stufe 3 die Katalysatorenperlen einer schonenden Trocknung durch 10- stündiges Überleiten von Luft bei einer Temperatur von 20 °C getrocknet. Bei dieser Massnahme tritt eine Schrumpfung und Verfestigung der Katalysatorenperlen auf einen Durchmesser von etwa 1 mm ein.

Es werden danach in Stufe 4 zur Äquilibrierung die Katalysatorperlen in eine 0,1 molare Na-Phosphat-Puffer-Lösung mit pH = 8 für einen Zeitraum von etwa 60 min. eingeführt. Die feuchten Biokatalystorperlen besitzen im Endzustand einen Durchmesser von 1,2 mm. Es ist also eine geringe Nachquellung eingetreten. Die Beladung an BFM beträgt 0,8 g/BFM/g Biokatalysator. Unter BFM ist die Biofeuchtmasse zu verstehen. Die Restaktivität betrug 30 %. Wenn eine Perlbildung durch Eintropfen einer Chitosanacetat-Lösung in eine Glutardialdehyd-Lösung erfolgt, so führt diese Arbeitsweise nur zu Biokatalysatoren mit einer geringen Beladung.

Beispiel 2

Es wird die Mischung (A) durch Suspension von 2.6 g Bovista plumbea NRLL 3824 in Form der zentrifugierten feuchten Masse in 23 g einer 3.5 Gew.-%igen, wässrigen Chitosan-acetat-Lösung als Polyelectrolyt hergestellt. Als Fällungsbad (B) mit der Verbindung, die mehrwertige Ionen enthält, die dem Polyelektrolyten der Mischung (A) entgegengesetzt geladen sind, wird eine 0.07 molare K$_4$ (Fe(CN)$_6$)-Lösung verwendet.

Es wird dann in Stufe 1 die Mischung (A) in die Mischung (B) eingetropft, wobei perlförmige Teilchen mit einem Durchmesser von ca. 6-7 mm entstehen. Diese werden unter weiterem Rühren bei einer Verweilzeit von 40 min. verfestigt. Es werden danach in Stufe 2 die gebildeten Katalysatorperlen mit der eingeschlossenen enzymatisch aktiven Substanz abfiltriert und mit einer 5 %-igen wässrigen Glyzerin-Lösung gewaschen.

Es werden danach in Stufe 3 die Katalysatorperlen einer schonenden Trocknung durch 10-stündiges Überleiten von Luft bei einer Temperatur von 16 °C unterzogen.

Hierbei tritt eine Schrumpfung und Verfestigung der Katalysatorperlen auf einen Durchmesser von etwa 2 mm ein. Es werden danach in Stufe 4 zur Äquilibrierung die Katalysatorperlen in eine 0.1 molare Na-Phosphat-Puffer-Lösung mit pH 7.8 für einen Zeitraum von etwa 4 h gegeben.

Die feuchten Biokatalysatoren besitzen im Endzustand einen Durchmesser von ca. 2.4-2.6 mm.

Die Beladung an BFM beträgt 0.93 g BFM/g Biokatalysator. Bei der Umsetzung von Penicillin V betrug die relative Aktivität 53 %.

Auch bei dieser besonders hohen Beladung an BFM wurden für verschiedene Katalysatorperlen aus diesem Beispiel hohe mechanische Festigkeiten im Bereich von 500 bis 800 p/Perle gemessen.

Das Verfahren der Erfindung bietet auch den Vorteil einer großen Breite in der Variation der Auswahl der Polymerkomponente und des Vernetzers.

Es kann somit das ionotrope Gel den physiologischen Eigenschaften der Biomasse angepaßt werden. Die Breite der Variation ergibt sich auch aus der Möglichkeit des Einsatzes ganzer Zellen, von Zellfragmenten und Enzymen als Biomasse. Es besteht dabei der Vorteil einer hohen Standzeit des Biokatalysators nach dem Verfahren der Erfindung.

Die Polymerkomponente ist technisch leicht zugänglich und preiswert. Ein weiterer technischer und wirtschaftlicher Vorteil des Verfahrens der Erfindung liegt auch darin, daß die Vernetzungsreaktion zur Bildung ionotroper Gele reversibel ist. Es läßt sich die Polymerkomponente des Biokatalysators nach dessen Verwendung in technischen Prozessen durch Auflösung des Neztwerkes wiedergewinnen.

Eine wesentliche Verbesserung durch das Verfahren der Erfindung liegt darin, daß erstmals die ionotrope Gelbildung mit kationischen Polyelektrolyten erfunden wurde. Somit ist es möglich, mit diesen Biokatalysatoren in den sehr häufig für biochemische Umsetzungen notwendigen Phosphat-Puffer-Lösungen zu arbeiten.

**Ansprüche**

1. Verfahren zur Herstellung von Biokatalysatoren mit hoher mechanischer Festigkeit und hoher Beladung an enzymatisch aktiver Substanz durch Polymereinschluss von Biomasse unter Verwendung der Mischung (A) aus einer Suspension oder Lösung einer enzymatisch aktiven Substanz in der wässrigen Lösung eines Polyelektrolyten, dadurch gekennzeichnet, dass die Konzentration des Polyelektrolyten im Bereich von 0,5 bis 15 Gew.-% liegt und als Fällungsbad (B) die Lösung des Salzes eines mehrwertigen, dem Polyelektrolyten der Mischung (A) engegengesetzt geladenen, Ions in einem Konzentrationsbereich von 0,02 bis 35 Gew.-% verwendet wird, und in Stufe 1 die Mischung (A) in die Mischung (B) eingedüst wird unter Bildung perlförmiger Teilchen eines Kornbereiches von etwa 0,5 bis etwa 10 mm, und diese unter Rühren in einer Verweilzeit zwischen etwa 0,5 bis 4 h verfestigt werden und in Stufe 2 die Katalysatorperlen mit der eingeschlossenen enzymatisch aktiven Substanz abfiltriert, mit Wasser oder einer physiologischen Kochsalzlösung oder der wässrigen Lösung oder Emulsion eines Schutzkolloids gewaschen und in Stufe 3 durch Überleiten von Luft einer Temperatur bis 80 °C bis etwa 48 h unter Schrumpfung getrocknet werden und in Stufe 4 die Katalysatorperlen in eine, das Enzym-System und das Netzwerk stabilisierende wässrige Lösung oder Emulsion eines Schutzkolloides eingebracht und danach im feuchten Zustand bis nach etwa 24 h ausgeführt werden.

2. Verfahren nach Anspruch 1, dadurch gekennzeichnet, dass in Mischung (A) als Polyelektrolyt ein modifiziertes Chitin in einem Konzentrationsbereich von 0,1 bis 15 Gew.-% eingesetzt wird.

3. Verfahren nach Anspruch 1, dadurch gekennzeichnet, dass in Mischung (A) als Polyelektrolyt ein protonisiertes Chitosan als positiv geladener Polyelektrolyt in einem Konzentrationsbereich von 0,1 bis 15 Gew.-% eingesetzt wird.

4. Verfahren nach den Ansprüchen 1 bis 3, dadurch gekennzeichnet, dass in der Mischung (B) als ionische Verbindung

K$_4$ (Fe(CN)$_6$)-Lösung und/oder
K$_3$ (Fe(CN)$_6$)-Lösung

eingesetzt wird.

5. Verfahren nach den Ansprüchen 1 bis 3, dadurch gekennzeichnet, dass zur Vernetzung des kationischen Polyelektrolyten in Mischung (A) das Polymere eines negativ geladenen Elektrolyten eingesetzt wird.

6. Verfahren nach den Ansprüchen 1 bis 3, dadurch gekennzeichnet, dass zur Vernetzung des kationischen Polyelektrolyten in Mischung (A) ein Polyphosphat mit einem Polymergrad kleiner 15 eingesetzt wird.

7. Verfahren nach den Ansprüchen 1 bis 3, dadurch gekennzeichnet, dass zur Vernetzung des kationischen Polyelektrolyten in Mischung (A) ein partiell verestertes Polyphosphat mt einem Polymergrad kleiner 15, verestert mit Alkohol oder Phenol, eingesetzt wird.

8. Verfahren nach den Ansprüchen 1 bis 7, dadurch gekennzeichnet, dass die enzymatisch aktive Substanz aus vermehrungsfähigen ganzen Zellen oder nicht vermehrungsfähigen ganzen Zellen, oder aus Zellfragmenten besteht und als Suspension eingesetzt wird.

9. Verfahren nach den Ansprüchen 1 bis 7, dadurch gekennzeichnet, dass die enzymatisch aktive Substanz aus einem isolierten Enzym oder einem Enzym-Komplex besteht und als Suspension eingesetzt wird.

10. Verfahren nach den Ansprüchen 1 bis 9, dadurch gekennzeichnet, das in den Stufen 2 und 4 als wässrige Lösung oder Emulsion eines Schutzkolloides eine Glycerinlösung mit einem Gehalt zwischen 1 und 20 Gew.-% verwendet wird.

11. Biokatalysatorperlen, erhältlich nach den Ansprüchen 1 bis 10.

## Claims

1. A method for producing biocatalysts having high mechanical strength and heavily loaded with enzymatically active substance by polymeric inclusion of biomass, by using the mixture (A) of a suspension or solution of an enzymatically active substance in an aqueous solution of a polyelectrolyte, characterized in that the concentration of the polyelectrolyte lies in a range between 0.5 to 15 % by weight, and the dissolution of a salt having polyvalent ions charged oppositely to the polyelectrolytes in a concentration range of 0.02 to 35 % by weight is used as a precipitation bath (B), and in stage 1, the mixture (A) being injected into the mixture (B) by forming beadlike particles in a grain-size range of about 0.5 to about 10 mm, these being solidified by slow, uniform stirring for about 0.5 to 4 h, and in stage 2, the catalyst beads thus formed being filtered out with the enclosed enzymatically active substance, being washed with a physiological salt solution or the aqueous solution or emulsion of a protective colloid, and in stage 3, by passing thereover air at a temperature of up to 80° for up to about 48 h, being dried by shrinking, and in stage 4, the catalyst beads being introduced into an aqueous solution or emulsion of a protective colloid, thus hardening the encyme system and the network, and thereafter being discharged after about 24 h in the moist condition.

2. Method according to claim 1, characterized in that in mixture (A) a modified chitin in a concentration range of 0.1 to 15 % by weight is used as polyelectrolyte.

3. Method according to claim 1, characterized in that in mixture (A) a protonized chitosan as a positively loaded polyelectrolyte in a concentration range of 0.1 to 15 % by weight is used as polyelectrolyte.

4. Method according to claims 1 through 3, characterized in that in the mixture (B)

$K_4$ (Fe(CN)$_6$)-solution and/or
$K_3$ (Fe(CN)$_6$)-solution

is used as ionic compound.

5. Method according to claims 1 through 3, characterized in that the polymere of a negative loaded electrolyte is used for interlacing of the cationic polyelectrolyte in mixture (A).

6. Method according to claims 1 through 3, characterized in that a polyphosphate with a polymer degree below 15 is used for interlacing of the cationic polyelectrolyte in mixture (A).

7. Method according to claims 1 through 3, characterized in that a partial esterified polyphosphate with a polymer degree below 15, esterified with alcohol or phenol, is used for interlacing of the cationic polyelectrolyte in mixture (A).

8. Method according to claims 1 through 7, characterized in that the enzymatically active substance consists of whole cells capable of reproduction or of cell fragments, and used in the form of a suspension.

9. Method according to claims 1 through 7, characterized in that the enzymatically active substance consists of an isolated enzyme or an enzyme-complex, and used in the form of a suspension.

10. Method according to claims 1 through 9, characterized in that in stages 2 and 4 a glyceric solution with a content between 1 and 20 % by weight is used as an aqueous solution or emulsion of a protective colloid.

11. Biocatalyst beads, available according to claims 1 through 10.

## Revendications

1. Procédé pour la fabrication des biocatalyseurs avec haute résistance mécanique et haut chargement de la substance enzyme-actif par inclusion polymère de biomasse en utilisant le mélange (A) d'une suspension ou solution d'une substance enzyme-actif dans une solution aqueuse d'un polyélectrolyte, caractérisé en ce que la concentration du polyélectrolyte se trouve dans la gamme de 0,5 à 15 % en poids et qu'on met en œuvre comme bain de précipitation (B) la solution du sel d'un ion plus valé qu'est chargé opposé au polyélectrolyte du mélange (A) dans une gamme de concentration de 0,02 à 35 % en poids et, dans étape 1, on injecte le mélange (A) dans le mélange (B) en formant des particules perlées d'une gamme de grain de 0,5 à 10 mm environ, ceux-ci en remuant sont solidifiés dans un temps de séjour entre 0,5 à 4 h environ, et dans étape 2, on filtre les perles du catalyseur avec la substance enzyme-actif inclus, lave avec l'eau ou une solution commune ou avec la solution aqueuse ou émulsion d'un colloïde protégé, et dans étape 3, on les sèche en passant de l'air d'une température jusqu'à 80 °C, jusqu'à 48 h environ en concentration, et dans étape 4, on met les perles du catalyseur dans une solution aqueuse ou émulsion d'un colloïde protégé, laquelle stabilise le gaz-enzyme-système, et l'entrelacs, qu'on décharge après jusqu'à 24 h environ à l'état humide.

2. Procédé suivant la revendication 1, caractérisé en ce qu'on met en œuvre dans le mélange (A) comme polyélectrolyte un chitin modifié dans une gamme de concentration de 0,1 à 15 % en poids.

3. Procédé suivant la revendication 1, caractérisé en ce qu'on met en œuvre dans le mélange (A) comme polyélectrolyte in chitan protonisé comme polyélectrolyte chargé positif dans une gamme de concentration de 0,1 à 15 % en poids.

4. Procédé suivant l'une ou l'autre des revendications 1 à 3, caractérisé en ce qu'on met en œuvre dans le mélange (B) comme composé ionique

K$_4$ (Fe(CN)$_6$)-solution et/ou
K$_3$ (Fe(CN)$_6$)-solution.

5. Procédé suivant l'une ou l'autre des revendications 1 à 3, caractérisé en ce qu'on met en œuvre dans le mélange (A) le polymère d'un électrolyte chargé négatif pour mettre en réseau du polyélectrolyte cationique.

6. Procédé suivant l'une ou l'autre des revendications 1 à 3, caractérisé en ce qu'on met en œuvre dans le mélange (A) un polyphosphate avec un degré polymère plus petit que 15 pour mettre en réseau du polyélectrolyte cationique.

7. Procédé suivant l'une ou l'autre des revendications 1 à 3, caractérisé en ce qu'on met en œuvre un polyphosphate estérifié partiel avec un degré polymère plus petit que 15, estérifié avec alcool ou phénol, pour mettre en réseau du polyélectrolyte cationique.

8. Procédé suivant l'une ou l'autre des revendications 1 à 7, caractérisé en ce que la substance enzyme-actif consiste de cellules entières, capable à propager ou non capable à propager ou de fragments de cellule, et mis en œuvre comme suspension.

9. Procédé suivant l'une ou l'autre des revendications 1 à 7, caractérisé en ce que la substance enzyme-actif consiste d'un enzyme isolé ou un enzyme-complex, et mis en œuvre comme suspension.

10. Procédé suivant l'une ou l'autre des revendications 1 à 9, caractérisé en ce qu'on utilise dans les étapes 2 et 4 comme solution aqueuse ou émulsion d'un colloïde protégé une solution de glycérine avec une teneur entre 1 et 20 % en poids.

11. On obtient perles du biocatalyseur suivant les revendications 1 à 10.